# EUROPÄISCHE PATENTSCHRIFT

(11) **EP 0 845 993 B1**
(45) Veröffentlichungstag und Bekanntmachung des Hinweises auf die Patenterteilung: **28.11.2001**
(21) Anmeldenummer: 96927629.4
(22) Anmeldetag: 30.07.1996
(51) Int. Cl.: A61K 38/16, A61K 38/49

(54) **VERWENDUNG VON PLASMINOGENAKTIVATOREN ZUM LOKALEN KNOCHENAUFBAU**
USE OF PLASMINOGENE ACTIVATORS TO STIMULATE LOCAL BONE GROWTH
UTILISATION D'ACTIVATEURS DE PLASMINOGENE POUR L'OSTEOGENESE LOCALE

(30) Priorität: 09.08.1995 DE 19529223
(43) Veröffentlichungstag der Anmeldung: 10.06.1998
(73) Patentinhaber: Roche Diagnostics GmbH, 68305 Mannheim (DE)
(72) Erfinder: HONOLD, Konrad, D-82377 Penzberg (DE); KLING, Lothar, D-68167 Mannheim (DE); LANG, Kurt, D-82377 Penzberg (DE); LESER, Ulrike, D-80796 München (DE)
(86) Internationale Anmeldenummer: EP9603345
(87) Internationale Veröffentlichungsnummer: WO9705891

(56) Entgegenhaltungen:
- EP-A- 0 227 400
- EP-A- 0 261 599
- EP-A- 0 297 860
- EP-A- 0 318 801
- WO-A-94/07537
- WO-A-94/15653
- US-A- 5 290 692
- US-A- 5 491 082
- DATABASE BIOSIS FILE SERVER STN KARLSRUHE ZUSAMMENFASSUNG 84:252958, HAJDUK ET AL: "THE EFFECT OF STREPTASE TRASKOLAN AND SEFRIL ON THE DEVELOPMENT OF EXPERIMENTAL OSTEITIS 2. ASSESSMENT OF MORPHOLOGICAL CHANGES IN THE INTERNAL ORGANS" XP002018215
- DATABASE MEDLINE FILE SERVER STN KARLSRUHE ZUSAMMENFASSUNG 82105103, HAJDUK ET AL: "EFFECT OF STREPTOKINASE,TRASKOLAN AND SEFRIL ON THE DEVELOPMENT OF EXPERIMENTAL OSTEOMYELITIS.I.EVALUATION OF LOCAL BONE CHANGES." XP002018216

## Beschreibung

Die Erfindung betrifft die Verwendung von Plasminogenaktivatoren zur Herstellung von Arzneimitteln zum lokalen Knochenaufbau sowie pharmazeutische Zusammensetzungen, die für diese Applikation geeignet sind. Der lokale Aufbau von Knochenmasse ist beispielsweise zur Heilung von Knochenbrüchen, bei tumorbedingtem Knochenabbau, bei kosmetischen Operationen, bei der Implantation von Zahnersatzmaterialien oder Knochenersatzmaterialen, wie künstliche Hüften oder Kniegelenke, oder zur Unterstützung des Einwachsens von Hilfsmaterialien, wie Nägel und Schrauben, erforderlich.

Es ist bekannt, daß Wachstumsfaktoren, wie transforming growth factor-β (TGF-β), basic fibroblast growth factor (bFGF) und platelet derived growth factor (PDGF) die Osteogenese positiv beeinflussen können (Pierce et al., J. Cell. Biol. 109 (1989) 429 - 440).

Aufgabe der Erfindung ist es, weitere Mittel bereitszustellen, welche, lokal appliziert, das Knochenwachstum bzw. die Knochenheilung unterstützen (Osteogenese).

Gegenstand der Erfindung ist die Verwendung von Plasminogenaktivatoren zur Herstellung eines Arzneimittels zur lokalen Osteogenese. Vorzugsweise wird der Plasminogenaktivator als Lösung (Injektion oder Infusion) oder in trägergebundener Form lokal an die zu behandelnde Stelle im Körper appliziert.

Unter einem Plasminogenaktivator ist ein Proteinenzym zu verstehen, welche das Zymogen Plasminogen durch Spaltung zwischen Arginin 506 und Valin 561 aktiviert, wobei die Serinprotease Plasmin entsteht. Plasmin kann eine Vielzahl von Proteinen, u. a. Fibrin, spalten. Plasminogenaktivatoren sind beispielsweise Urokinase (uPA), tissue plasminogen activator (t-PA) und Streptokinase. Urokinase besteht aus 411 Aminosäuren, die in drei Domänen (epidermal growth factor like domain (EGF), Kringel-Domäne und Serinproteasedomäne) eingeteilt werden kann. Urokinase wurde bereits 1982 gereinigt und charakterisiert. Die rekombinante Herstellung wurde von Holz, W.E. et al., Biotechnology 3 (1985) 923 - 929 beschrieben.

t-PA ist ein Protein, welches aus 527 Aminosäuren besteht. Es enthält ebenfalls verschiedene Domänen, die als finger region, EGF-like Domäne, 2-Kringel-Region und Serinprotease-Region bezeichnet werden. t-PA wurde gereinigt und charakterisiert von Collen, D. et al., Thromb. Haemostas. 43 (1980) 77 - 89. Die rekombinante Herstellung ist in Pennica, D. et al., Nature 301 (1983) 214 - 220 beschrieben. Derivate und Muteine von Urokinase und t-PA sind in Harris T.J.R., Protein Engineering 1 (1987) 449 - 458 beschrieben.

Vorzugsweise werden Plasminogenaktivatoren verwendet, welche analoge Eigenschaften wie humaner t-PA, insbesondere bezüglich plasminogenolytischen und immunologischen Eigenschaften sowie bezüglich der Fibrinbindung, besitzen.

Besonders bevorzugt werden Plasminogenaktivatoren verwendet, die eine geringe Fibrinbindung zeigen. Solche Plasminogenaktivatoren sind beispielsweise in der WO 90/09437 beschrieben und bestehen vorzugsweise aus Kringel 2 und der Proteasendomäne von t-PA.

Die Applikation des Plasminogenaktivators an die zu behandelnde Stelle im Körper, vorzugsweise am Knochen, kann entweder in Lösung zweckmäßig durch Infusion oder Injektion (vorzugsweise lokale Injektion) oder trägergebunden erfolgen. Trägergebundene Plasminogenaktivatoren können beispielsweise als Gele, Pasten oder als Beschichtung auf Implantaten appliziert werden.

Als Träger werden biokompatible und vorzugsweise bioabbaubare Materialen verwendet. Vorzugsweise induzieren die Materialien selbst zusätzlich noch die Wundheilung oder die Osteogenese.

Zur Applikation ist es bevorzugt, den Plasminogenaktivator in polymere Gele oder Filme einzubetten, dadurch zu immobilisieren und diese Präparation direkt auf die zu behandelnde Stelle am Knochen aufzutragen. Derartige polymere Basisgele oder Filme bestehen beispielsweise im wesentlichen aus Glycerin, Methylcellulose, Hyaluronsäure, Polyethylenoxiden und/oder Polyoxameren. Ebenfalls geeignet sind Kollagen, Gelatine und Alginate und sind beispielsweise in WO 93/00050 und WO 93/20859 beschrieben. Weitere Polymere sind Polymilchsäure (PLA) und Copolymere aus Milchsäure und Glykolsäure (PLPG) (Hollinger et al., J. Biomed. Mater. Res. 17 (1983) 71-82) sowie das Knochenderivat "Demineralized Bone Matrix" (DBM) (Guterman et al., Kollagen Rel. Res. 8 (1988) 419 - 431). Ebenfalls geeignet sind Polymere, wie sie beispielsweise zur Adsorption für TGF-β verwendet werden und in der EP-A 0 616 814 und der EP-A 0 567 391 beschrieben sind und synthetische Knochenmatrices gemäß WO 91/18558.

Ebenso geeignet als Träger für den Plasminogenaktivator sind Materialien, die üblicherweise bei der Implantation von Knochenersatzstoffen oder von sonstigen therapeutischen Wirkstoffen verwendet werden. Solche Träger basieren beispielsweise auch auf Calciumsulfat, Tricalciumphosphat, Hydroxyapatit und Polyanhydriden. Außer diesen bioabbaubaren Trägern sind auch Träger geeignet, die nicht bioabbaubar sind, aber biokompatibel sind. Solche Träger sind beispielsweise gesinterter Hydroxyapatit, Bioglas, Aluminate oder andere keramische Materialien (z. B. Calcium-Aluminat-Phosphat). Diese Materialien werden bevorzugt in Kombination mit den bioabbaubaren Materialien, wie insbesondere Polymilchsäure, Hydroxyapatit, Collagen oder Tricalciumphosphat angewendet. Weitere nicht abbaubare Polymere sind beispielsweise im US-Patent 4,164,560 beschrieben.

Besonders bevorzugt ist es, den Plasminogenaktivator kontinuierlich in kleiner Dosis am Wirkort freizusetzen. Vorzugsweise wird hierzu trägergebundener Plasminogenaktivator verwendet. Hierfür besonders geeignet sind "slow release pellets" von Innovative Research of America, Toledo, Ohio, USA. Besonders bevorzugt werden Pellets verwendet, welche den Plasminogenaktivator über mehrere Tage, vorzugsweise bis zu 100 Tagen, bei einer täglichen Dosis von 1 - 10 mg/kg pro Tag, freisetzen.

Die Dosierung der Plasminogenaktivatoren für den erfindungsgemäßen Verwendungszweck liegt bei Applikation in Lösung zweckmäßig zwischen 0,1 µg/ml und 100 µg/ml. Die Menge an applizierter Lösung hängt hauptsächlich von der Größe der zu behandelnden Knochenstelle ab. Üblicherweise werden jedoch 1 ml pro Injektion gegeben. Die Anzahl der Applikationen ist abhängig von der Prognose und von jedem Fachmann feststellbar.

Zur Applikation in Lösung, können die Plasminogenaktivatoren in Wasser, Polyethylenglykol, Propylenglykol, Ethanol, natürlichen Ölen, wie Pflanzenölen, Benzylalkohol, Natriumchlorid und/oder verschiedenen Puffern gelöst werden. Andere Adjuvantien, die dem Fachmann bekannt sind, können ebenfalls verwendet werden.

Die folgenden Beispiele und Abbildungen erläutern die Erfindung weiter. Die Beispiele sind als Hilfsmittel zur Ausführung der Erfindung zu verstehen, die im Rahmen der Lehre der Erfindung variiert werden können.
- Fig. 1A: zeigt den Einfluß von r-PA (t-PA Derivat aus Kringel 2 und Protease) auf die Knochendichte (c = Kontrollösung (100 %)).
- Fig. 1B: zeigt den Einfluß von Plasmin auf die Knochendichte (c = Kontrollösung (100 %)).
- Fig. 2: zeigt den Einfluß von r-PA und t-PA auf die Knochendichte (c = Kontrollösung (100%)).
- Fig. 3: zeigt den Einfluß der Proteasedomäne von t-PA auf die Knochendichte (c = Kontrollösung 100 %)

### Beispiel 1

### Herstellung von immobilisiertem Plasminogenaktivator

TCP (Tricalciumphosphat, Partikelgröße 150 - 250 µm, hergestellt nach WO 94/15653) werden zur Entpyrogenisierung mit 0,5 mol/l NaOH vorbehandelt. Die Beschichtung mit Plasminogenaktivator erfolgt durch Inkubation der Partikel mit einer Lösung von 1 - 1000 mg/l Plasminogenaktivator in 50 mmol/l Arginin/HCl, pH 7 - 7,4.

Die Adsorption des Plasminogenaktivators an die Keramik-Partikel wurde durch UV-Absorptionsmessung der Lösung vor und nach Inkubation mit den Partikeln bestimmt und beträgt ca. 1,5 µg Plasminogenaktivator/mg Keramikpartikel.

Die beschichteten Keramiken wurden mit Wasser und PBS für jeweils 30 Minuten gewaschen. Die Überprüfung der Beschichtung erfolgt, indem die gewaschenen Keramiken mit einer Lösung von 0,4 mol/l Natriumphosphatpuffer, pH 6 - 8 inkubiert werden. Im Überstand kann vom Träger eluierter Plasminogenaktivator nachgewiesen werden. Ebenso kann die enzymatische/proteolytische Aktivität des immobilisierten Plasminogenaktivators nachgewiesen werden.

### 2. Knochenbildung in vivo durch Plasminogenaktivator

Der Nachweis der Knochenbildung wird in einem Versuchsmodell nach Mackie und Trechsel, Bone 11 (1990) 295 - 300 durchgeführt.

### 2.1 Behandlung der Tiere

### 2.1.1 Plasmin und r-PA

Weibliche, 7 Wochen alte geschlechtsreife Balb/C-Mäuse (Gewicht ca. 20 g) wurden willkürlich in Gruppen zu je 6 Tieren eingeteilt. Vier Gruppen erhielten unterschiedliche Dosen Plasmin oder Plasminogenaktivator r-PA (rekombinantes Derivat von t-PA (tissue plasminogen activator) aus den Domänen K2 und P, Herstellung nach WO 90/09437) appliziert, eine Gruppe diente als Kontrolle und erhielt den Lösungspuffer ohne Plasminogenaktivator mit Rinderserumalbumin (RSA) als Neutralprotein. Jedem Tier wurden einmal täglich 50 µl der entsprechenden Lösung mittels einer Hamilton-Spritze direkt unter die Kopfhaut auf die Kalotte appliziert. Die Applikationsdauer betrug 2 x 5 Tage, mit einer zweitägigen Pause zwischen den beiden Applikationsperioden.

Verwendet wurden Lösungen von Plasmin in PBS, das 1 mg/ml Rinderserumalbumin (BSA) enthielt in den Konzentrationen 0,001 U/ml, 0,01 U/ml, 0,1 U/ml und 1 U/ml oder r-PA in PBS/1 mg/ml BSA in den Konzentrationen 0,1 mg/l, 1 mg/l, 10 mg/l und 100 mg/l. Aliquote wurden bei -20°C gelagert und jeweils unmittelbar vor der Verabreichung aufgetaut. Die Tagesdosen betrugen bei Plasmin 5 x 10⁻⁵ U, 5 x 10⁻⁴ U, 5 x 10⁻³ U und 5 x 10⁻² U pro Tier, bei r-PA, 0,005 µg, 0,05 µg, 0,5 µg und 5 µg pro Tier. Die Kalottenentnahme erfolgte am 26. Versuchstag (= 14. Tag nach letzter Applikation).

### 2.1.2 r-PA und t-PA

t-PA (human tissue plasminogen activator) wurde rekombinant hergestellt in CHO-Zellen gemäß EP-B 0 093 619. Die Applikation von t-PA und r-PA erfolgte wie in 2.1.1 beschrieben. Als Dosis wurden jeweils 5 µg/Tier (100 mg/l) verwendet. Als Kontrollösung wurden 150 mmol/l Phosphatpuffer, pH 6,0, 10 mmol/l Tranexamsäure, 7 mg/ml Saccharose, 0,01 % Tween® 80 verwendet. Die Applikationsdauer betrug 4 x 5 Tage mit einer zweitägigen Pause zwischen den Applikationsperioden. Die Kalottenentnahme erfolgte am 40. Versuchstag (=14. Tag nach letzter Applikation).

### 2.1.3 Proteasedomäne des t-PAs

Die Proteasedomäne wurde gemäß WO 96/17928 in E.coli-Zellen rekombinant hergestellt. Die Applikation erfolgte wie in 2.1.1 beschrieben. Als Dosis wurden jeweils 1,15 µg/Tier (23 mg/l) oder 3,80 µg/Tier (76 mg/l) verwendet. Die verwendete Kontrollösung war identisch zu der in Abschnitt 2.1.2 beschriebenen. Die Applikationsdauer betrug 4 x 5 Tage mit einer zweitägigen Pause zwischen den Applikationsperioden. Die Kalottenentnahme erfolgte am 40. Versuchstag.

### 2.2 Gewebepräparation

Die Tiere wurden durch Halswirbeldislokation getötet und die Kalotten herauspräpariert. Aus jeder Kalotte wurde ein trapezförmiges Stück herausgestanzt, das im wesentlichen aus dem rechten und linken Os parietale und Os frontale besteht. Die Stanzstücke wurden 24 Stunden lang bei 4°C in 50 %igem Ethanol + 1 % CaCl₂ fixiert und danach zur Aufbewahrung in 70%iges Ethanol überführt.

### 2.3 Röntgenanalyse

Die Kalottenstücke wurden aus der Ethanollösung genommen und für 30 Minuten an der Luft getrocknet.

Von allen Kalottenstücken wurde eine gemeinsame Röntgenaufnahme gemacht (Röntgenplatte: Structurix D4pDW, 13 x 18 cm/100, Fa. AGFA-GAEVERT; Röntgengerät: Torrex 120, Fa. EG&E, Astrophysics Research Corporation, 35 kV, 5 mA, 40 sec). Anschließend wurde die Röntgendichte (Grauwert der Kalotten mit Hilfe eines Echtfarbenanalysegerätes (CBA 800, Fa. Wild-Leitz) in einem bestimmten, immer gleichen Meßfenster quantifiziert. Dabei deckte das Meßfenster den größten Teil einer Kalottenhälfte ab (in der Regel der rechten), wobei die Suturen nicht im Meßbereich lagen. Die Meßwerte jeder Dosisgruppe wurden gemittelt und zur Kontrolle (= 100%) in Beziehung gesetzt.

### 2.4 Ergebnisse

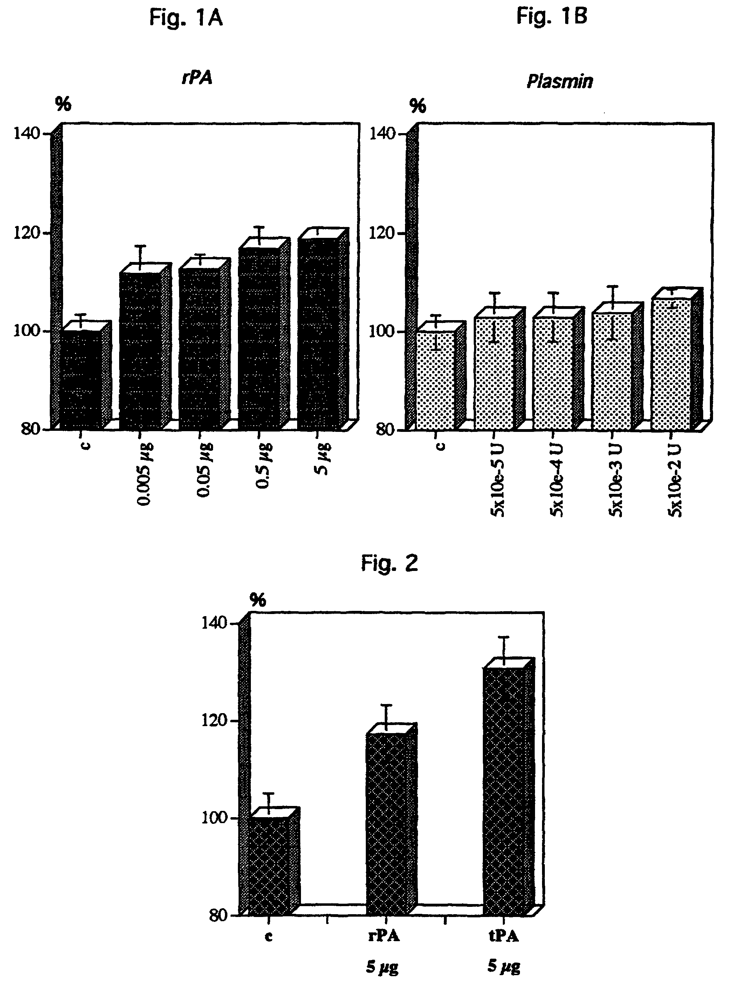

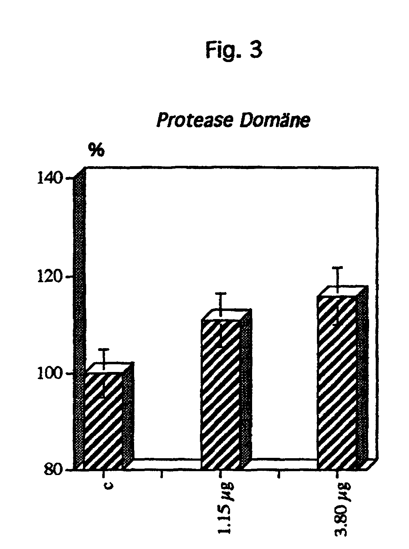

Fig. 1A + 1B zeigen die Ergebnisse der Röntgenanalyse aus 2.1.1. Danach hat Plasmin praktisch keinen Einfluß auf die Knochendichte, während der Plasminogenaktivator r-PA die Knochendichte deutlich erhöht. Fig. 2 zeigt die Ergebnisse aus Beispiel 2.1.2. Danach zeigt sich, daß sowohl r-PA als auch t-PA die Knochendichte deutlich erhöhen. Fig. 3 zeigt das Ergebnis aus Beispiel 2.1.3. Daraus ergibt sich, daß auch die Proteasedomäne des t-PAs die Knochendichte deutlich erhöht.

### Referenzliste

Collen, D. et al., Thromb. Haemostas. 43 (1980) 77 - 89

EP-A 0 567 391

EP-A 0 616 814

Guterman et al., Kollagen Rel. Res. 8 (1988) 419 - 431

Harris T.J.R., Protein Engineering 1 (1987) 449 - 458

Hollinger et al., J. Biomed. Mater. Res. 17 (1983) 71 - 82

Holz, W.E. et al., Biotechnology 3 (1985) 923- 929

Mackie und Trechsel, Bone 11 (1990) 295 - 300

Pennica, D. et al., Nature 301 (1983) 214 - 220

Pierce et al., J. Cell. Biol. 109 (1989) 429 - 440

US-P 4,164,560

WO 90/09437

WO 91/18558

WO 93/00050

WO 93/20859

WO 94/15653

WO 96/17928

## Patentansprüche

1. Verwendung eines Plasminogenaktivators zur Herstellung eines Arzneimittels zur lokalen Osteogenese.

2. Verwendung nach Anspruch 1, **dadurch gekennzeichnet, daß** der Plasminogenaktivator als Lösung an die zu behandelnde Stelle im Körper, durch Injektion oder Infusion, applizierbar ist.

3. Verwendung nach Anspruch 1, **dadurch gekennzeichnet, daß** der Plasminogenaktivator in trägergebundener Form lokal an die zu behandelnde Stelle im Körper applizierbar ist.

4. Verwendung nach Anspruch 1 oder 3, **dadurch gekennzeichnet, daß** der Plasminogenaktivator durch Mischung mit einem polymeren Gel immobilisiert wird.

5. Knochenimplantat, **dadurch gekennzeichnet, daß** es mit einem Plasminogenaktivator in Mischung mit einem polymeren Gel in immobilisierter Form oder mit einem Plasminogenaktivator in einer bioverträglichen, wasserunlöslichen Trägermatrix, welche den Plasminogenaktivator kontinuierlich freisetzt, beschichtet ist.

6. Knochenersatzstoff, **dadurch gekennzeichnet, daß** er einen Plasminogenaktivator in einer bioverträglichen, wasserunlöslichen Trägermatrix enthält, welche den Plasminogenaktivator kontinuierlich freisetzt.

## Claims

1. Use of a plasminogen activator to produce a pharmaceutical preparation for local osteogenesis.

2. Use as claimed in claim 1, **characterized in that** the plasminogen activator can be administered as a solution to the site in the body to be treated by injection or infusion.

3. Use as claimed in claim 1, **characterized in that** the plasminogen activator can be administered locally to the site in the body to be treated in a carrier-bound form.

4. Use as claimed in claim 1 or 3, **characterized in that** the plasminogen activator is immobilized by mixing with a polymeric gel.

5. Bone implant, **characterized in that** it is coated with a plasminogen activtor in a mixture with a polymeric gel in an immobilized form or with a plasminogen activator in a biocompatible, water-insoluble carrier matrix which continuously released the plasminogen activator.

6. Bone substitute, **characterized in that** it contains a plasminogen activator in a biocompatible, water-insoluble carrier matrix which continuously releases the plasminogen activator.

## Revendications

1. Utilisation d'un activateur du plasminogène pour la fabrication d'un médicament destiné à l'ostéogenèse locale.

2. Utilisation selon la revendication 1, **caractérisée en ce que** l'activateur du plasminogène peut être administré sous forme de solution sur les zones à traiter dans le corps, par injection ou perfusion.

3. Utilisation selon la revendication 1, **caractérisée en ce que** l'activateur du plasminogène peut être administré sous forme fixée à un support, sur les zones à traiter du corps.

4. Utilisation selon la revendication 1 ou 3, **caractérisée en ce que** l'activateur du plasminogène est immobilisé par mélange avec un gel polymère.

5. Implant osseux, **caractérisé en ce qu'**il est revêtu avec un activateur du plasminogène sous forme immobilisée en mélange avec un gel polymère, ou avec un activateur du plasminogène dans une matière de support insoluble dans l'eau, biocompatible, qui libère en continu l'activateur du plasminogène.

6. Substitut osseux, **caractérisé en ce qu'**il contient un activateur du plasminogène dans une matière de support insoluble dans l'eau, biocompatible, qui libère en continu l'activateur du plasminogène.
